(19)

**Europäisches Patentamt**
**European Patent Office**
**Office européen des brevets**

(11) **EP 4 765 174 A1**

(12) **EUROPEAN PATENT APPLICATION**

(43) Date of publication:
**24.06.2026 Bulletin 2026/26**

(21) Application number: **25220828.5**

(22) Date of filing: **04.12.2025**

(51) International Patent Classification (IPC):
**H01F 27/40** (2006.01)   **H01F 3/14** (2006.01)
**H02M 3/28** (2006.01)

(52) Cooperative Patent Classification (CPC):
**H01F 27/40; H02M 3/285; H01F 3/14;**
**H01F 2038/006**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **11.12.2024 CN 202411824184**

(71) Applicant: **Shanghai United Imaging Healthcare Co., Ltd.**
**Shanghai 201807 (CN)**

(72) Inventors:
• **ZHU, Guoping**
  **Shanghai, 201807 (CN)**
• **LUO, Biao**
  **Shanghai, 201807 (CN)**
• **WU, Jinglin**
  **Shanghai, 201807 (CN)**

(74) Representative: **Murgitroyd & Company**
**165-169 Scotland Street**
**Glasgow G5 8PL (GB)**

(54) **TRANSFORMER, POWER CONVERSION DEVICE, AND MEDICAL DEVICE**

(57)   Disclosed are a transformer, a power conversion device, and a medical device. The transformer includes a primary winding, a plurality of magnetic core assemblies, and a plurality of secondary windings. The primary winding is configured to pass through the plurality of magnetic core assemblies. The plurality of secondary windings are wound on the plurality of magnetic core assemblies, respectively. Each of the plurality of magnetic core assemblies has an air gap of the same size. And/or, the transformer includes a capacitor and an impedance element. The capacitor is connected in series with the impedance element. The series-connected capacitor and impedance element are connected to two ends of a corresponding secondary winding.

FIG. 23

**Description**

TECHNICAL FIELD

The present disclosure relates to the field of transformers, particular to a transformer, a power conversion device, and a medical device.

BACKGROUND

High-voltage power supplies are widely used in fields such as medical X-ray generation, air pollution control, industrial testing, high-energy physics, and plasma applications, with their core being high-voltage high-frequency transformers. However, conventional centralized magnetic core high-voltage high-frequency transformers suffer from problems such as large volume caused by excessively high voltage gain, large parasitic capacitance of windings, voltage imbalance distribution inside windings, internal partial discharge, and difficult heat dissipation.

SUMMARY

In a first aspect, an embodiment of the present disclosure provides a transformer. The transformer includes a primary winding, a plurality of magnetic core assemblies, and a plurality of secondary windings. The primary winding is configured to pass through the plurality of magnetic core assemblies. The plurality of secondary windings are wound on the plurality of magnetic core assemblies, respectively. Each of the plurality of magnetic core assemblies has an air gap of the same size. And/or, the transformer includes a capacitor and an impedance element. The capacitor is connected in series with the impedance element. The series-connected capacitor and impedance element are connected to two ends of a corresponding secondary winding.

In some embodiments, the impedance element includes a resistor.

In some embodiments, the impedance element includes a resistor and an inductor, and the resistor is connected in parallel with the inductor.

In some embodiments, the impedance element includes an inductor.

In some embodiments, corresponding to the limitation of each of the plurality of magnetic core assemblies having the air gap of the same size, the transformer includes a capacitor, and two ends of the capacitor are connected to two ends of a corresponding secondary winding.

In some embodiments, the transformer includes a plurality of rectifier circuits, and the plurality of rectifier circuits are connected to the plurality of secondary windings, respectively.

In some embodiments, parameters of the impedance element are determined based on at least one of parameters of: a frequency of an alternating current (AC) voltage inputted to the primary winding, a load range of the transformer, differences in characteristics of the magnetic core assemblies, or a preset loss of the transformer.

In some embodiments, each of the plurality of magnetic core assemblies is made of silicon steel, ferrite, or alloy.

In some embodiments, the transformer is configured for a high-voltage power supply.

In some embodiments, the air gap has the same length.

In some embodiments, winding directions of the secondary windings are the same, and numbers of turns of the secondary windings are the same.

In some embodiments, a cross-section of each magnetic core assembly is in a toroidal shape, a rectangle shape, or a triangle shape.

In some embodiments, the differences in the characteristics of the magnetic core assemblies include the differences in permeabilities and cross-sectional areas of magnetic cores.

In some embodiments, a capacitance value of the capacitor is determined based on at least one of parameters of: a frequency of an AC voltage inputted to the primary winding, a load range of the transformer, differences in characteristics of the magnetic core assemblies, or a preset loss of the transformer.

In a second aspect, an embodiment of the present disclosure provides a power conversion device. The power conversion device includes an inverter circuit and the transformer according to the first aspect. The inverter circuit is connected to the transformer.

The inverter circuit is connected to the primary winding of the transformer.

In some embodiments, the inverter circuit includes a switching element; and the switching element is configured to adjust the direction of current in the inverter circuit by changing an on state or an off state of the switching element.

In some embodiments, the inverter circuit is configured to use a pulse frequency modulation.

In a third aspect, a medical device is provided in an embodiment of the present disclosure. The medical device includes the transformer according to the first aspect.

In some embodiments, the medical device includes an X-ray machine, a CT scanner, or a radiotherapy machine.

BRIEF DESCRIPTION OF THE DRAWINGS

FIG. 1 is a schematic structural diagram of a centralized magnetic core high-voltage high-frequency transformer in the related art.

FIG. 2 is a schematic structural diagram of a distributed high-voltage high-frequency transformer in the related art.

FIG. 3 is an equivalent circuit diagram of a distributed high-voltage high-frequency transformer in the related art.

FIG. 4 is a two-stage equivalent circuit diagram of a distributed high-voltage high-frequency transformer in the related art.

FIG. 5 is a diagram showing magnitude-frequency characteristic curves of a voltage difference transfer function $H_{\Delta 12\_origin(s)}$ in the related art.

FIG. 6 is a schematic structural diagram of a distributed high-voltage high-frequency transformer according to an embodiment of the present application, with parallel capacitors balancing voltage.

FIG. 7 is an equivalent circuit diagram of a distributed high-voltage high-frequency transformer according to an embodiment of the present application, with parallel capacitors sharing voltage.

FIG. 8 is a second-order equivalent circuit diagram of a distributed high-voltage high-frequency transformer according to an embodiment of the present application, with parallel capacitors for balancing voltage.

FIG. 9 is a diagram showing magnitude-frequency characteristic curves of a voltage difference transfer function $H_{\Delta 12\_cp(s)}$ under heavy load conditions with parallel voltage-balancing capacitors according to an embodiment of the present application.

FIG. 10 is a diagram showing magnitude-frequency characteristic curves of a voltage difference transfer function $H_{\Delta 12\_cp(s)}$ under light load conditions with parallel voltage-balancing capacitors according to an embodiment of the present application.

FIG. 11 is a schematic diagram of a magnetic core assembly with an air gap according to an embodiment of the present application.

FIG. 12 is a comparison diagram of magnetization curves of a toroidal magnetic core before and after introducing an air gap according to an embodiment of the present application.

FIG. 13 is a second-order equivalent circuit diagram of a transformer having a magnetic core assembly with an air gap according to an embodiment of the present application.

FIG. 14 is a schematic structural diagram of a transformer with capacitors connected in series to resistors, respectively, according to an embodiment of the present application.

FIG. 15 is an equivalent circuit diagram of a transformer with capacitors connected in series to resistors according to an embodiment of the present application.

FIG. 16 is a second-order equivalent circuit diagram of a transformer with capacitors connected in series with resistors, respectively according to an embodiment of the present application.

FIG. 17 is a diagram showing magnitude-frequency characteristic curves of a voltage difference transfer function $H_{\Delta 12\_cp+Rd(s)}$ according to an embodiment of the present application.

FIG. 18 is a diagram showing magnitude-frequency characteristic curves of the voltage difference transfer function $H_{\Delta 12\_cp+Rd(s)}$ according to another embodiment of the present application.

FIG. 19 is a schematic structural diagram of a transformer with resistors connected to inductors in parallel according to an embodiment of the present application.

FIG. 20 is a schematic structural diagram of a transformer with resistors connected in series to inductors respectively according to an embodiment of the present application.

FIG. 21 is a schematic diagram of a transformer having a magnetic core assembly with an air gap according to an embodiment of the present application.

FIG. 22 is a schematic diagram of a transformer having a magnetic core assembly with an air gap and a secondary winding connected to capacitors in parallel according to an embodiment of the present application.

FIG. 23 is a schematic diagram of a transformer having a magnetic core assembly with an air gap and a secondary winding connected to a capacitor and a resistor in parallel according to an embodiment of the present application.

FIG. 24 is a block diagram showing a power conversion device according to an embodiment of the present application.

DETAILED DESCRIPTION OF THE EMBODIMENTS

To make the objectives, technical solutions and advantages thereof in the present disclosure clearer, the present disclosure will be further described in detail below with reference to the accompanying drawings and embodiments. It should be understood that the specific embodiments described herein are only used to explain the present disclosure and are not used to limit the present disclosure.

In conventional technology, the high-voltage side windings (i.e., secondary windings) of distributed high-voltage high-frequency transformers are respectively wound on different magnetic cores. The secondary windings are arranged in series after rectification and filtering to obtain high-voltage direct current output. While, primary windings pass through all magnetic cores. This multi-magnetic-core distributed high-voltage transformer has a compact structure, good heat dissipation, and small parasitic capacitance of windings, making the transformer fairly suitable for high-voltage high-frequency applications. However, when the characteristics of the magnetic cores differ from each other, for example, the permeabilities of the magnetic cores are different or the cross-sectional areas of the magnetic cores are different, the output voltages from the high-voltage side windings will be inconsistent, thus causing a voltage breakdown of partial winding with an excessively high voltage or an overvoltage damage to the corresponding rectifier circuit in extreme cases.

Accordingly, it is necessary to address the above technical problem by providing a transformer, a power conversion device, and a medical device that can address the problem of the voltage imbalance of the secondary windings of the transformer.

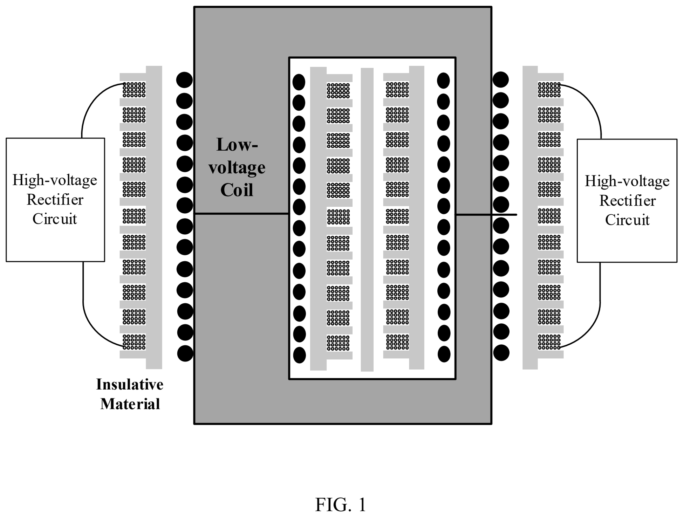

Most high-voltage high-frequency transformers in the related art have a centralized magnetic core structure, where both primary and secondary windings are wound on the same magnetic circuit. FIG. 1 is a schematic structural diagram of a centralized magnetic core high-voltage high-frequency transformer in the related art. As shown in FIG. 1, a low-voltage coil of a transformer is wound around a magnetic core. Moreover, the high-voltage high-frequency transformer is provided with a large number of high-voltage side windings to achieve a high-voltage output. The large number of high-voltage side windings will lead to serious problems of parasitic capacitance of the windings and voltage sharing. The high-voltage side windings may be damaged due to partial discharge after a long-term use. In addition, since the ground potential of the magnetic core, the voltage between the primary winding and the secondary winding is relatively high, to achieve insulation between the primary winding and the secondary winding, a large amount of insulative material is required between the primary winding and the secondary winding, and inside the secondary winding, which will seriously deteriorate the heat dissipation of the high-voltage side windings, and the high-voltage side windings may be damaged due to overheating under a high power.

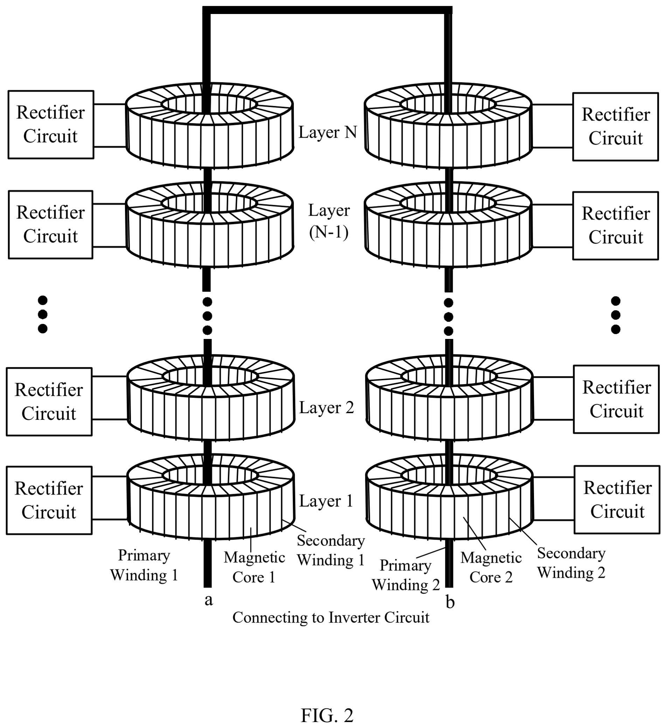

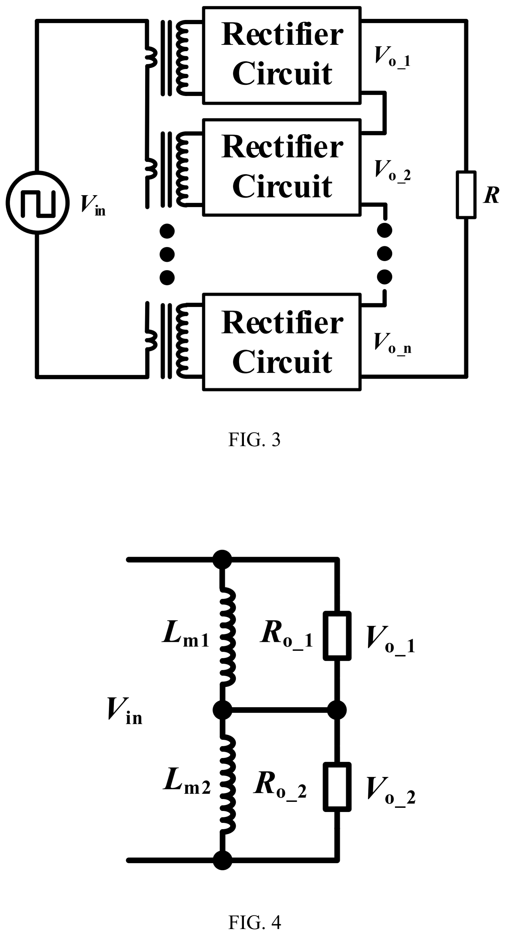

FIG. 2 is a schematic structural diagram of a distributed high-voltage high-frequency transformer in the related art. As shown in FIG. 2, the primary winding passes through all magnetic cores and is connected to an inverter circuit. The high-voltage side windings (secondary windings) are wound on different magnetic cores, respectively. After each of the secondary windings is connected to a rectifier circuit, the secondary windings are connected in series to obtain a high-voltage direct current output. As shown in FIG. 2, 2N secondary windings are provided, where N is a positive integer. In the structure of this distributed high-voltage transformer, the turns ratio of the high-voltage side windings to the primary winding can be greatly reduced, which can greatly alleviate or even avoid the problems of parasitic capacitance and partial discharge of the high-voltage side windings. In addition, in the structure of the distributed high-voltage transformer, the problems of heat dissipation, insulation, and electric field gradient in the transformer can be greatly improved. However, due to possible differences of up to about 30% in the permeability and equivalent cross-sectional area of the magnetic circuit of the magnetic cores around which each of the secondary windings is wound, secondary voltages of the secondary windings will be different. FIG. 3 is an equivalent circuit diagram of a distributed high-voltage high-frequency transformer in the related art. As shown in FIG. 3, in extreme cases, a partial breakdown may occur to a winding with excessively high voltage or an overvoltage damage may be caused to the corresponding rectifier circuit due to excessive differences in the secondary voltages. In FIG. 3, $V_{in}$ represents an input voltage of the primary winding, $V_{o\_n}$ represents the output voltage corresponding to the $N$-th secondary winding in the transformer, and R represents the equivalent resistance of the load of the N secondary windings.

Taking a two-stage distributed high-voltage high-frequency transformer as an example, FIG. 4 is a two-stage equivalent circuit diagram of a distributed high-voltage high-frequency transformer in the related art. As shown in FIG. 4, the transfer function $H_{\Delta 12\_origin}(s)$ of the voltage difference $\Delta V_{12}$ between $V_{o\_1}$ and $V_{o\_2}$, and $V_{in}$ is as follows:

$$H_{\Delta 12\_origin}(s) = \frac{2sL_{m1}L_{m2}(R_{O_1}+R_{O_2})+(L_{m1}-L_{m2})R_{O\_1}R_{O\_2}}{2sL_{m1}L_{m2}(R_{O_1}+R_{O_2})+(L_{m1}+L_{m2})R_{O\_1}R_{O\_2}} \qquad (1)$$

where, $V_{o\_1}$ represents the secondary voltage value corresponding to a secondary winding in the transformer, $R_{o\_1}$ represents the equivalent resistance value of the circuit load corresponding to the secondary winding in the transformer, and $L_{m1}$ represents the equivalent inductance value of the circuit corresponding to the secondary winding in the transformer. $V_{o\_2}$ represents the secondary voltage value corresponding to an other secondary winding in the transformer, $R_{o\_2}$ represents the equivalent resistance value of the circuit load corresponding to the other secondary winding in the transformer, $L_{m2}$ represents the equivalent inductance value of the circuit corresponding to the other secondary winding in the transformer, and s represents the number of turns of the secondary winding.

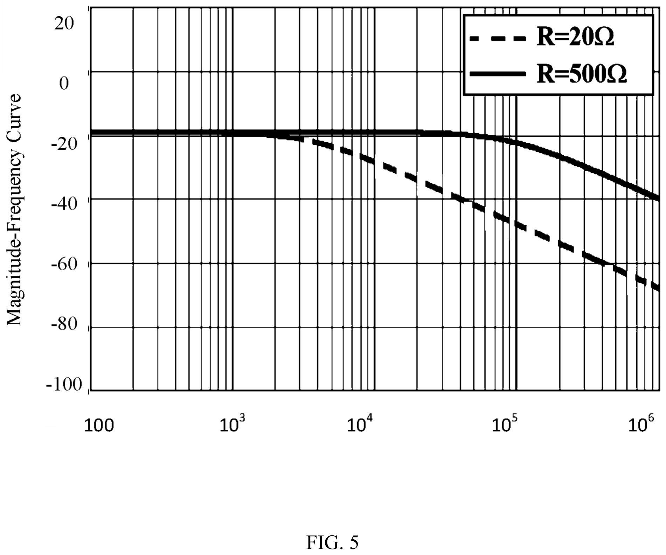

In an embodiment, $L_{m1}$=1000uH, $L_{m2}$=800uH. In a case of heavy load that $R_{o\_1}$=$R_{o\_2}$=20Ω, and in a case of light load that $R_{o\_1}$=$R_{o\_2}$=500Ω and magnitude-frequency characteristic curves diagram of the voltage difference transfer function HΔ12_origin(s) are shown in FIG. 5. In FIG. 5, the abscissa represents a frequency and the ordinate represents a voltage difference. As can be seen from FIG. 5, as the frequency increases, the voltage difference gradually decreases. The reason is that the higher the frequency is, the larger the inductive reactance is. A voltage sharing ratio is mainly determined by the load resistance. In another aspect, in the middle frequency band, the larger the load resistance is, the more slowly the voltage difference attenuates. The above two points mean that voltage imbalances of the secondary windings are more likely to occur under light load or no load conditions, and the degree of voltage imbalance is mainly determined by the difference in magnetizing inductance. The greater the difference in magnetic cores, the higher the degree of the voltage imbalance.

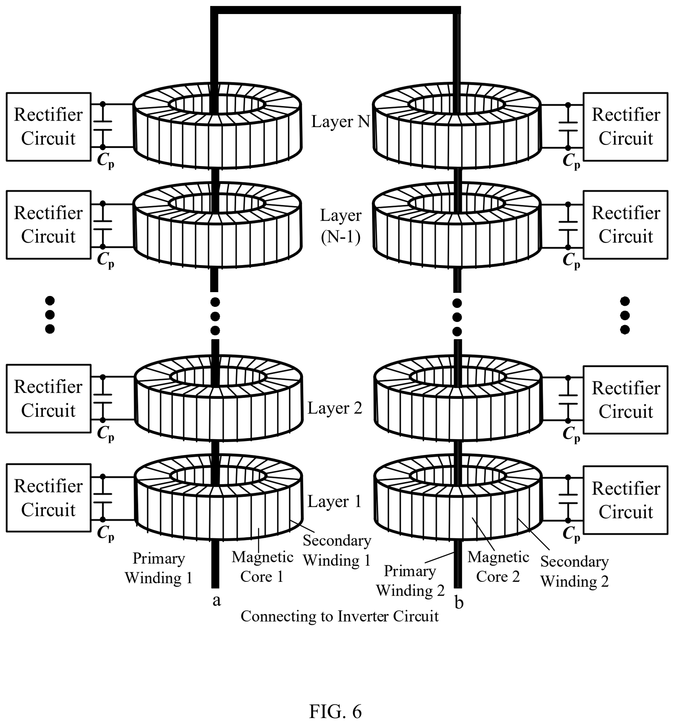

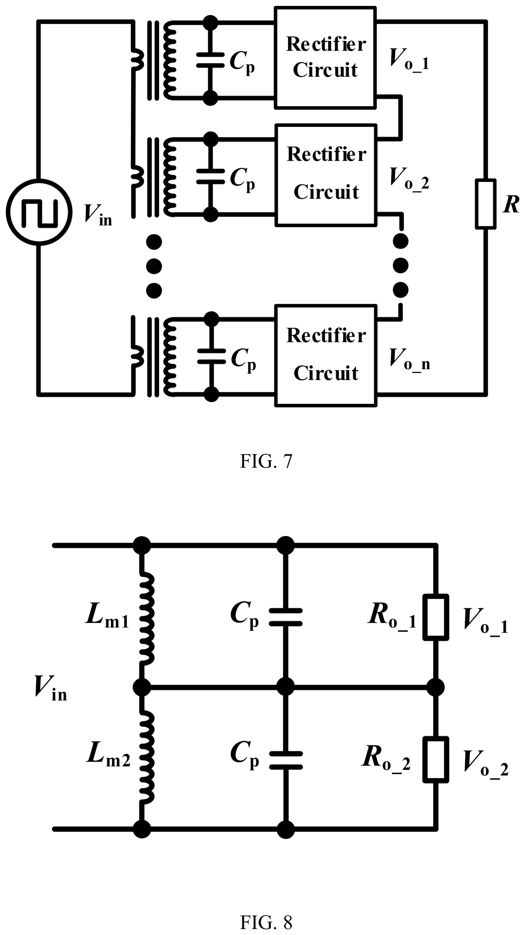

To address the problem of voltage imbalance of the high-voltage side windings of the transformer, voltage-balancing capacitors $Cp$ can be connected in parallel with the high-voltage side windings to compensate for the differences in the characteristics of respective magnetic cores and achieve voltage balance of the high-voltage side windings. FIG. 6 shows a schematic structural diagram of a distributed high-voltage high-frequency transformer with parallel capacitors $Cp$ for balancing voltage. Connecting voltage-balancing capacitors $Cp$ in parallel with the high-voltage side windings can compensate for the differences in the characteristics of the respective magnetic cores and achieve voltage balancing of the high-voltage side windings. In an embodiment, as shown in FIG. 7, an equivalent circuit of a distributed high-voltage high-frequency transformer using parallel capacitors $Cp$ for balancing voltage is shown.

Taking a two-stage distributed high-voltage high-frequency transformer as an example, FIG. 8 is a second-order equivalent circuit diagram of a distributed high-voltage high-frequency transformer with parallel capacitors Cp for balancing voltage. As shown in FIG. 8, in an embodiment, $R_{o\_1}=R_{o\_2}=R$, the transfer function $H_{\Delta12\_cp}(s)$ of the voltage difference $\Delta V_{12}$ between $V_{o\_1}$ and $V_{o\_2}$, and $V_{in}$ is as follows:

$$H_{\Delta12_{Cp}}(s) = \frac{L_{m1}-L_{m2}}{2s^2 L_{m1} L_{m2} C_p + \frac{2s L_{m1} L_{m2}}{R} + L_{m1} + L_{m2}} \qquad (2)$$

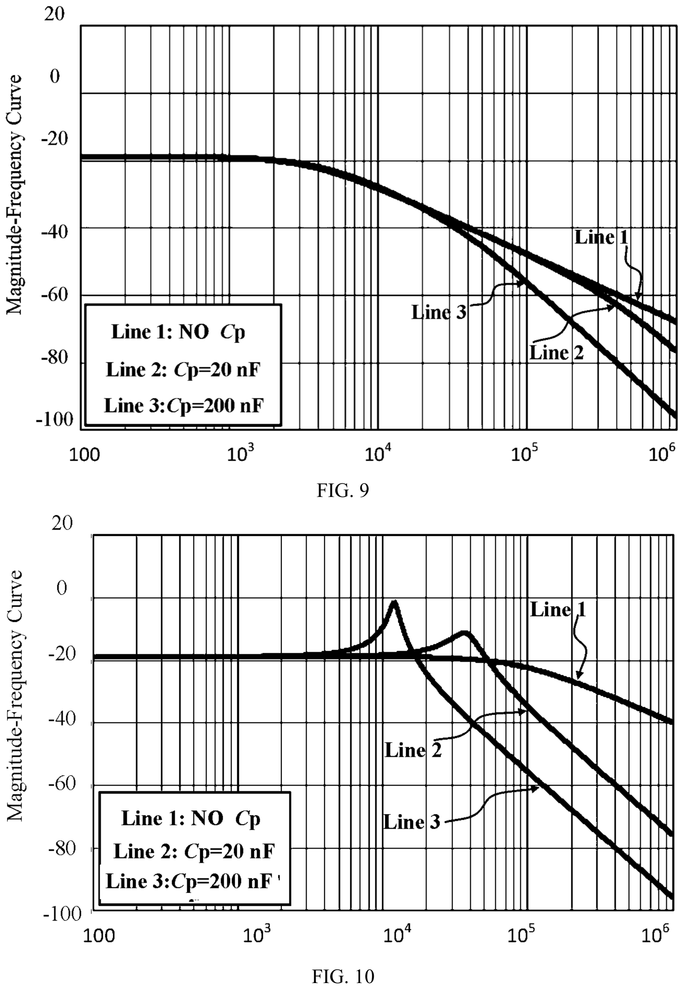

Taking $L_{m1}$=1000uH, $L_{m2}$=800uH, and $R_{o\_1}=R_{o\_2}$=20Ω under heavy load condition and $R_{o\_1}=R_{o\_2}$=500Ω under light load as an example, FIG. 9 is a diagram showing magnitude-frequency characteristic curves of the voltage difference transfer function $H_{\Delta12\_cp}(s)$ under heavy load conditions with parallel voltage-balancing capacitors Cp, and FIG. 10 is a diagram showing magnitude-frequency characteristic curves of the voltage difference transfer function HΔ12_cp(s) under light load conditions with parallel voltage-balancing capacitors Cp. Specifically, FIG. 9 shows a magnitude-frequency characteristic curve of the transformer under heavy load conditions without parallel voltage-balancing capacitor $Cp$, and the magnitude-frequency characteristic curves of the transformer under heavy load conditions with parallel voltage-balancing capacitors $Cp$, in which the capacitances of the parallel voltage-balancing capacitors are 20nF and 200nF, respectively. FIG. 10 shows a magnitude-frequency characteristic curve of the transformer under light load conditions without parallel voltage-balancing capacitor $Cp$, and the magnitude-frequency characteristic curves of the transformer under heavy load conditions with parallel voltage-balancing capacitors $Cp$, in which the capacitances of the parallel voltage-balancing capacitors are 20nF and 200nF, respectively. It can be seen from FIG. 9 and FIG. 10 that the voltage sharing ratio of the transformer under heavy load conditions is mainly determined by the equivalent load resistance. The existence of the parallel voltage-balancing capacitor $Cp$ basically has no impact on the degree of voltage imbalance. However, when the transformer is under light load, the existence of the parallel voltage-balancing capacitor $Cp$ can significantly reduce the degree of voltage imbalance of the secondary winding in the high-frequency band, but in some of the middle frequency band, the parallel capacitor $Cp$ may instead amplify the degree of voltage imbalance of the secondary winding.

Based on the structure shown in FIG. 6, when a pulse frequency modulation is used in the inverter circuit, the switching frequency of the input voltage changes in a wide range. When the capacitance value of the parallel voltage-balancing capacitor $Cp$ is inappropriate, the voltage balance of the secondary winding can be improved in some frequency band of the transformer, while the voltage balance of the secondary winding can be deteriorated in some frequency band. Therefore, when the input voltage is within a certain frequency range, an appropriate parallel voltage-balancing capacitor $Cp$ can surely improve the voltage balance of the winding. However, when the value of the parallel voltage-balancing capacitor $Cp$ is inappropriate, the voltage imbalance of the high-voltage side windings will be amplified. In addition, the existence of the parallel voltage-balancing capacitor $Cp$ leads to an increase in reactive power circulation inside a power supply, reducing the efficiency of the power supply. In another aspect, a parasitic parallel capacitor having large capacitance affects the working state of the front-end inverter circuit and restricts the improvement of the switching frequency of the inverter circuit.

Based on the above problems, a transformer is provided in an embodiment of the present disclosure. The transformer includes a primary winding, a plurality of magnetic core assemblies, and a plurality of secondary windings. The primary winding passes through the plurality of magnetic core assemblies. The plurality of secondary windings is wound on the plurality of magnetic core assemblies, respectively.

Each of the magnetic core assembly is configured to concentrate and guide the magnetic field in the transformer, and each magnetic core assembly can be made of a material such as silicon steel, ferrite, or alloy. The magnetic core assembly can be either closed or have an air gap. The primary winding can also be referred to the primary side winding, and the secondary winding can also be referred to the secondary side winding. The primary winding and the secondary winding belong to the circuit of the transformer and are wound by wires having relatively high conductivity. The primary winding and the secondary winding are further explained below.

The primary winding is configured to receive an input electrical signal. When an alternating current passes through the primary winding, an alternating magnetic field is generated in the magnetic core assembly, thereby transmitting electrical power to the secondary winding. The primary winding is a coil made of wires. The primary winding can be a single-turn coil or a multi-turn coil obtained by winding. In an embodiment, the primary winding can be wound on one or more magnetic cores to change the distribution of the alternating magnetic field.

The secondary winding is configured to output an electrical signal to a load. When the alternating magnetic field is generated in the magnetic core assembly, the secondary winding generates an electromotive force and thus an electric potential, providing an electrical power to an external load. The secondary winding is also a coil made of wires. The magnetic core assemblies and the secondary windings are in one-to-one correspondence. The secondary windings are wound on the magnetic core assemblies, respectively. The winding directions and the numbers of turns of the secondary windings can be the same or different, and are not limited herein and may be determined according to actual requirements.

To address the problem of voltage imbalance of the secondary windings of the transformer, the transformer further includes a capacitor and an impedance element. The capacitor is connected in series with the impedance element. The series-connected capacitor and impedance element are connected to the two ends of a corresponding secondary winding. Namely, the series-connected capacitor and impedance element form an integrated whole, and the two ends of the integrated whole are connected to the two ends of the corresponding secondary winding.

The impedance element includes a resistor, an inductor, a capacitor, or a component composed of these basic elements such the resistor, the inductor, and the capacitor. In an embodiment, the series-connected capacitor and impedance element are connected between the two ends of each of the secondary windings. By connecting the impedance element in series with the capacitor, the secondary voltage of the secondary winding can be redistributed based on the impedance, thereby mitigating the problem of voltage imbalance of the secondary windings caused by different characteristics of the magnetic core assemblies.

Alternatively, to address the problem of voltage imbalance of the secondary windings of the transformer, each of the magnetic core assemblies has an air gap of the same size.

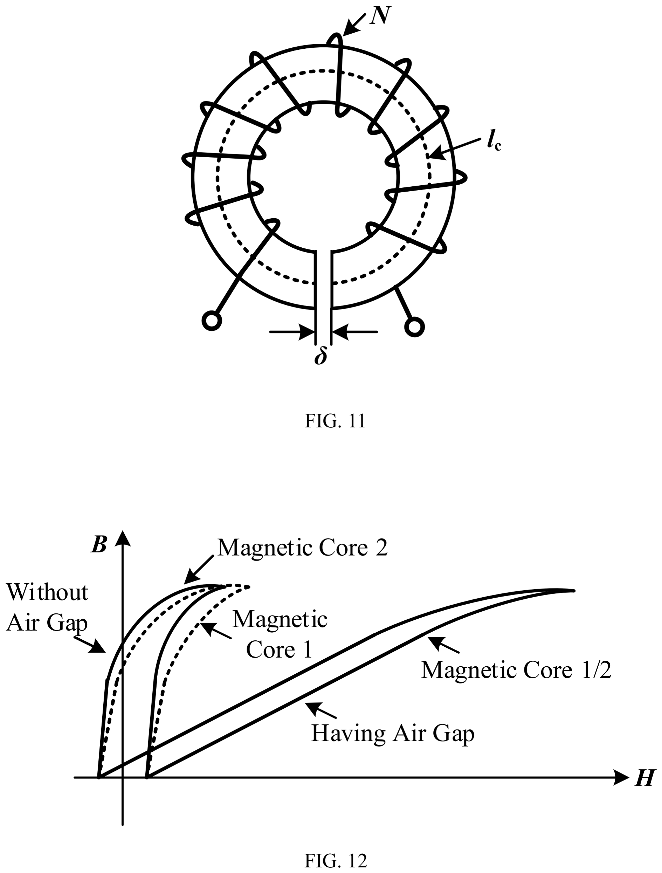

FIG. 11 shows a magnetic core assembly having an air gap. The cross-section of the magnetic core assembly can also be in any other shape besides the toroidal shape. For example, the cross-section of each magnetic core assembly may be in a rectangle shape, or a triangle shape, etc. FIG. 12 is a comparison diagram of magnetization curves of a toroidal magnetic core before and after an air gap is formed, where the abscissa refers to magnetization intensity and the ordinate refers to magnetic induction intensity. As shown in FIG. 12, the difference in permeability between the curves labeled with a Magnetic Core 1" and a Magnetic Core 2 is large when no air gap is formed. The difference in permeability between the curves labeled with the Magnetic Core 1 and the Magnetic Core 2 is small when the air gap is formed.

In an embodiment, the influence of the air gap on the characteristics of the magnetic core is as follows.

In an embodiment, a cross-sectional area of the magnetic core is *Ae,* the equivalent length of the magnetic circuit is *lc,* and the length of the air gap is $\delta$. The relative permeability of the Magnetic Core 1 is $\mu_{r1}$. The relative permeability of the Magnetic Core 2 is $\mu_{r2}$. For high-permeability magnetic cores, both $\mu_{r1}$ and $\mu_{r2}$ are much greater than 1. The permeability of the air gap is $\mu_0$. The equivalent permeabilities $\mu_{e1}$ and $\mu_{e2}$ of the Magnetic Core 1 and the Magnetic Core 2 after their respective air gaps are formed are determined by formula (3) and formula (4), respectively.

$$\mu_{e1} = \frac{1}{\frac{1}{\mu_{r1}} + \frac{\delta}{l_c}} \approx \frac{l_c}{\delta} \qquad (3)$$

$$\mu_{e2} = \frac{1}{\frac{1}{\mu_{r2}} + \frac{\delta}{l_c}} \approx \frac{l_c}{\delta} \qquad (4)$$

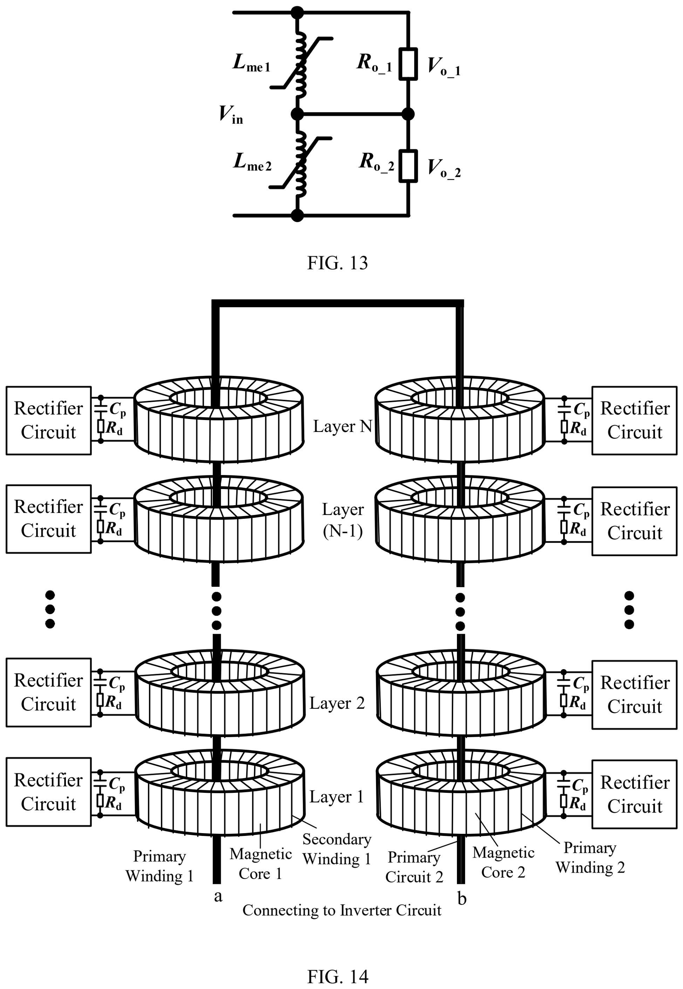

It can be known from the above formula (3) and formula (4) that the equivalent permeability of the magnetic core after the air gap is formed is mainly determined by the air gap. Therefore, as long as the lengths $\delta$ of the respective air gaps of the magnetic cores are the same, the influence of the difference in the relative permeability of the magnetic cores can be eliminated, thereby ensuring the excitation inductances of the respective magnetic cores to be the same. For ease of understanding, FIG. 13 is a second-order equivalent circuit diagram of a transformer having a magnetic core assembly with an air gap. As shown in FIG. 13, the transfer function $H_{\Delta 12\_gap}(s)$ of the voltage difference $\Delta V_{12}$ between $V_{o\_1}$ and $V_{o\_2}$, and $V_{in}$ is expressed by formula (5).

$$H_{\Delta 12\_gap}(s) = \frac{L_{me1} - L_{me2}}{\frac{sL_{me1}L_{me2}}{R} + L_{me1} + L_{me2}} \qquad (5)$$

It can be known from formula (5) that, by forming air gaps of the same size in the magnetic core assemblies, the excitation inductances can be consistent, and the voltage imbalance of the secondary windings can be eliminated.

Therefore, by forming air gaps of the same size in the respective magnetic cores, the permeabilities of the magnetic core assemblies can be changed, and the equivalent permeabilities of the respective magnetic cores can be made consistent, thereby compensating for the differences in the characteristics of the respective magnetic cores, and the excitation inductances can also be consistent, thereby ensuring the voltage balance of the secondary windings.

Alternatively, to address the problem of voltage imbalance of the secondary windings of the transformer, the transformer further includes a capacitor and an impedance element. The capacitor is connected in series with the impedance element. The series-connected capacitor and impedance element is an integrated whole, two ends of which are connected to the two ends of the corresponding secondary winding, respectively. Each of the magnetic core assemblies has an air gap of the same size. By connecting voltage-balancing capacitors $Cp$ in parallel with the secondary windings to compensate for the differences in the characteristics of each of the magnetic cores. The impedance element connected in series with the capacitor avoids a situation where the degree of voltage imbalance of the secondary windings is deteriorated due to inappropriate capacitance value of the capacitor. In addition, by forming the air gaps in the magnetic core assemblies, the permeabilities of the magnetic core assemblies are changed, and the differences in the characteristics of the magnetic cores are further compensated, thereby addressing the problem of voltage imbalance of the plurality of secondary windings of the transformer.

In this embodiment, by providing parallel capacitors to compensate for the differences in the characteristics of the respective magnetic cores, the voltage balance of the high-voltage side windings is achieved. Meanwhile, the parallel capacitor is connected to a damping resistor, to avoid the increase of the voltage imbalance of the high-voltage side windings caused by inappropriate capacitance value of the capacitor. Alternatively, each of the distributed magnetic cores has the air gap, the difference in the magnetic resistances of the magnetic circuits is improved through the air gap, to compensate for the differences in the characteristics of the magnetic cores, thereby achieving the voltage balance of the plurality of secondary windings.

In an embodiment, the transformer includes a plurality of rectifier circuits. The rectifier circuits are connected to the secondary windings, respectively. The rectifier circuit is configured to convert the alternating current input by the secondary winding into a direct current, thereby providing power for the load connected to the transformer. A conventional rectifier circuit can be used for rectification, and the selection of the rectifier circuit is not limited herein.

In an embodiment, parameters of the impedance element are determined based on at least one of the following parameters: the frequency of the alternating current (AC) voltage input to the primary winding, the load range of the transformer, the differences in the characteristics of the magnetic core assemblies, or a preset loss of the transformer.

When the frequency of the AC voltage is fixed, the greater the differences in the characteristics of the magnetic core assemblies, the larger the parameters values of the impedance element can be set. For example, a resistor with a larger resistance value or an inductor with a larger inductance value can be used. When the frequency of the AC voltage is fixed, the smaller the differences in characteristics of the magnetic core assemblies, the smaller the parameters values of the impedance element can be set. For example, a resistor with a smaller resistance value or an inductor with a smaller inductance value can be used.

If the load range of the transformer is small, the voltage imbalance caused by the differences in characteristics of the magnetic core assemblies is relatively severe, therefore the parameters values of the impedance element can be relatively large. If the load range of the transformer is large, the voltage imbalance caused by the differences in characteristics of the magnetic core assemblies is relatively small, therefore the parameters values of the impedance element can be relatively small.

The differences in characteristics of the magnetic core assemblies include, but are not limited to, differences in the permeabilities and the cross-sectional areas of the magnetic cores. The greater the differences among the magnetic core assemblies, the more severe the voltage imbalance caused, and the larger the values of the parameters of the impedance element can be set. The smaller the differences of the magnetic core assemblies, the slighter the voltage imbalance caused, and the smaller the parameters values of the impedance element can be set.

The smaller the parameters values of the impedance element are, the smaller the preset loss of the transformer is. The larger the parameters values of the impedance element are, the larger the preset loss of the transformer is.

In this embodiment, the parameters of the impedance element are determined based on at least one of the frequency of the AC voltage input to the primary winding, the load range of the transformer, the characteristics of the magnetic core assemblies, or the preset loss of the transformer, thereby guaranteeing the voltage balance of the plurality of secondary windings, and reducing the loss of the transformer.

In an embodiment, when a capacitor connected in series with a resistor is used to achieve the voltage balance, the capacitance value of the capacitor is also determined based on at least one of the following parameters: the frequency of the AC voltage input to the primary winding, the load range of the transformer, the differences in characteristics of the magnetic core assemblies, or the preset loss of the transformer. When the AC voltage input to the primary winding is generated based on the inverter circuit, the frequency of the AC voltage input to the primary winding falls within the frequency range of the switching element in the inverter circuit.

In an embodiment, the impedance element includes, but is not limited to, a resistor. Specifically, the capacitor is connected in series with a resistor, and the series-connected capacitor and resistor are an integrated whole. The two ends of the whole are connected to two ends of the corresponding secondary winding, respectively, so that the two ends of each of the secondary windings are connected with the series-connected capacitor and resistor. The capacitor can be connected in series with a single resistor, or the capacitor can be connected in series with a component composed of a plurality of resistors.

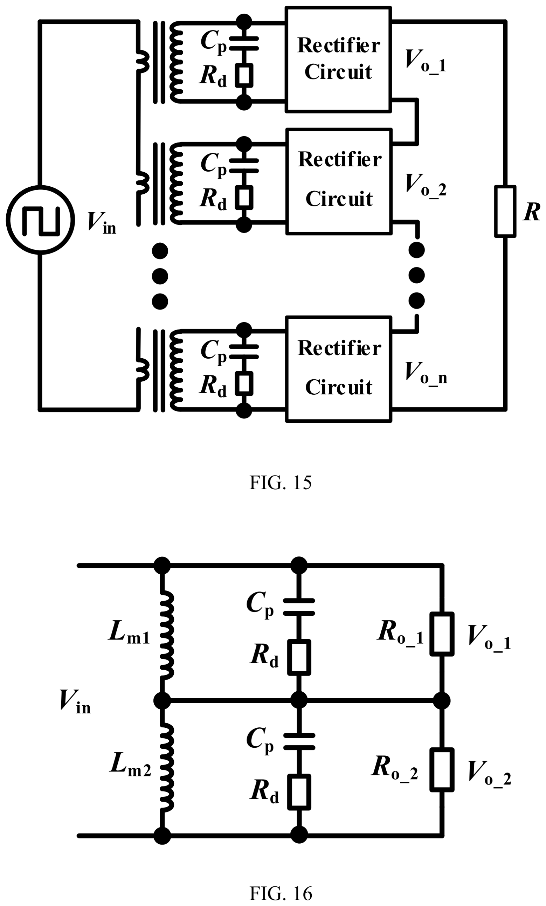

In an embodiment, the transformer includes a plurality of rectifier circuits. The plurality of rectifier circuits are connected to the secondary windings, respectively. A damping resistor $R_d$ is provided. FIG. 14 is a schematic structural diagram of a transformer with a capacitor connected in series with a resistor. When each of the secondary windings, the series-connected resistor and capacitor, and the rectifier circuit are connected in parallel, as shown in FIG. 14, the transformer includes N layers of secondary windings. Each layer includes two secondary windings and two corresponding magnetic core assemblies. In an embodiment, FIG. 15 shows an equivalent circuit of the transformer where each of the capacitors $C_P$ is connected in series with the resistor $R_d$. In an embodiment, taking a two-stage distributed high-voltage high-frequency transformer as an example, FIG. 16 is a second-order equivalent circuit diagram of the transformer with capacitors connected in series with resistors respectively. As shown in FIG. 16, $R_{o\_1}=R_{o\_1}=R$, the transfer function $H_{\Delta12\_cp+Rd}(s)$ of the voltage difference $\Delta V_{12}$ between $V_{o\_1}$ and $V_{o\_2}$, and $V_{in}$ is as follows:

$$
H_{\Delta12_{Cp}+Rd}(s) = \left[ R(L_{m1} - L_{m2})\left(s^2 C_p^2 R_d^2 + s C_p R_d + 1\right) \right] / \left\{ 2s^3 L_{m1} L_{m2} C_p^2 R_d (R_d + \right.
$$

$$
R) + 2s^2 \left[ L_{m1} L_{m2} C_p (2R_d + R) + (L_{m1} + L_{m2}) C_p^2 R_d^2 R + 2s \left[ L_{m1} L_{m2} + \right. \right.
$$

$$
\left. 2s(L_{m1} + L_{m2}) C_p R_d R \right] + R(L_{m1} + L_{m2}) \right] \} \tag{6}
$$

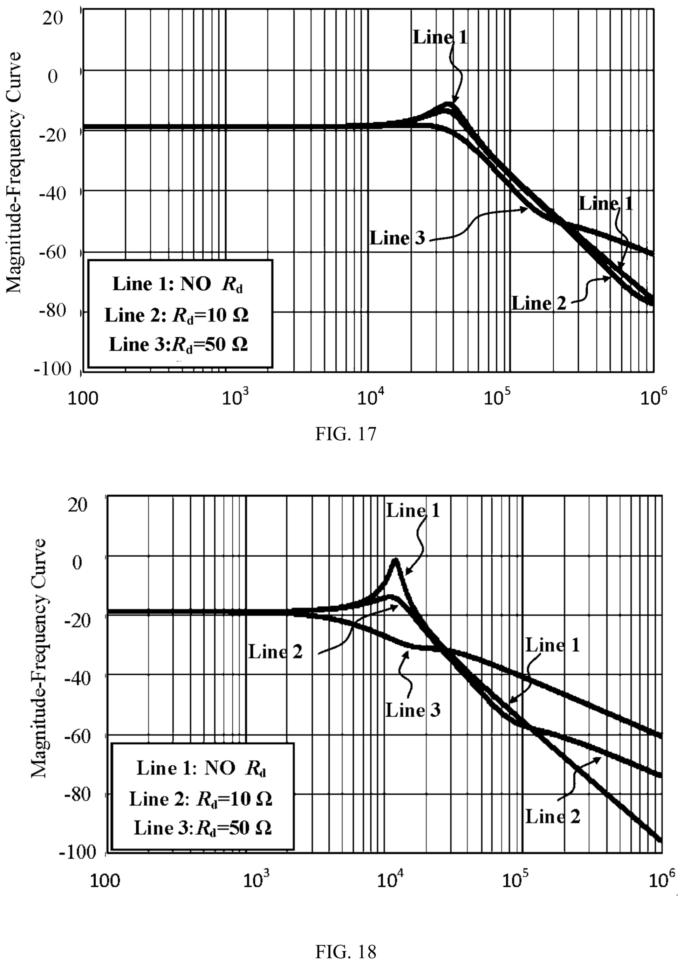

In an embodiment, $L_{m1}=1000uH$, $L_{m2}=800uH$, and $R_{o\_1}=R_{o\_1} = Ro_{\_2}=R$. FIG. 17 is a diagram showing magnitude-frequency characteristic curves of a voltage difference transfer function $H_{\Delta12\_cp+Rd(s)}$, and shows three magnitude-frequency characteristic curves of the voltage difference transfer function $H_{\Delta12\_cp+Rd}(s)$, and the parallel voltage-balancing capacitor $C_P$ is set to be 20 nF, and the series resistor $R_d$ is alternatively set to be 50 Ω or 10 Ω, or no series resistor $R_d$ is provided, and both the parallel voltage-balancing capacitor $C_P$ and the series resistor $R_d$ correspond to the voltage difference transfer function. In an embodiment, FIG. 18 is a diagram showing magnitude-frequency characteristic curves of the voltage difference transfer function $H_{\Delta12\_cp+Rd(s)}$ and shows, three magnitude-frequency characteristic curves of the voltage difference transfer function $H_{\Delta12\_cp+Rd}(s)$, and the parallel voltage-balancing capacitor $C_P$ is set to be 200nF, and the series resistor $R_d$ is alternatively set to be 50 Ω or 10 Ω, or no series resistor $R_d$ is provided, and both the parallel voltage-balancing capacitor $C_P$ and the series resistor $R_d$ correspond to the voltage difference transfer function.

Comparing the embodiments of FIG. 17 and FIG. 18 with the embodiment of FIG. 10, it is obvious that connecting an appropriate damping resistor $R_d$ in series with the voltage-balancing capacitor $C_P$ can not only significantly reduce the degree of the voltage imbalance in the high-frequency band, but also avoid the problem that the degree of the voltage imbalance of the secondary windings is amplified in part of the middle frequency band for the reason that only the parallel voltage-balancing capacitor $C_P$ is used when the transformer is under light load, , and the embodiment of connecting an appropriate damping resistor $R_d$ in series with the voltage-balancing capacitor $C_P$ is suitable for application scenarios of frequency conversion modulation.

In the embodiment, connecting the capacitors in parallel to the secondary windings, respectively, can compensate for the differences in the characteristics of the respective magnetic cores. By connecting the resistor in series with the capacitor, the problem that the voltage imbalance of the secondary windings deteriorates due to an inappropriate capacitance value can be avoided through the resistor.

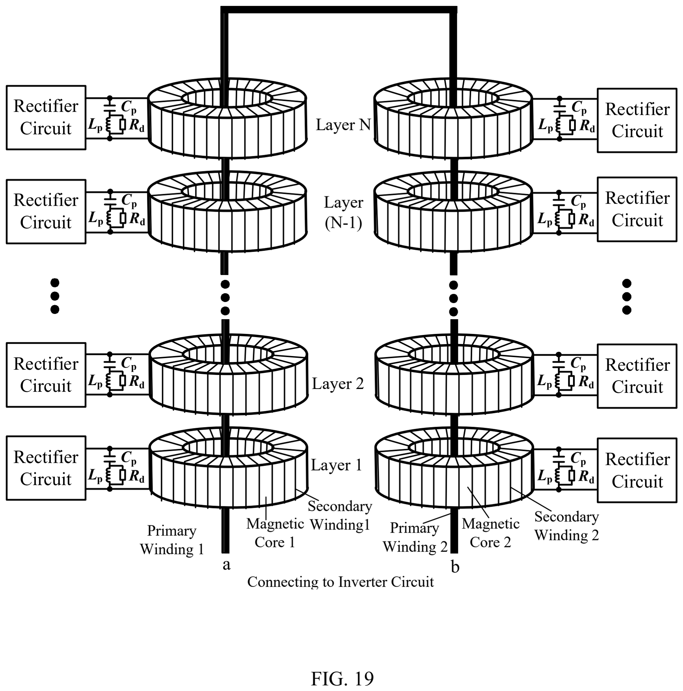

In an embodiment, the impedance element includes a resistor and an inductor. The resistor is connected in parallel with the inductor. The parallel-connected resistor and inductor can jointly determine the impedance of the circuit, thereby reducing the resistance value of the resistor required to address the problem of voltage imbalance. In addition, the lower the resistance value of the resistor, the smaller the loss of the resistance, so that the efficiency of the transformer can be improved. In an embodiment, the transformer includes a plurality of rectifier circuits. The circuits of the plurality of rectifiers are connected to the plurality of secondary windings, respectively. The impedance element includes the damping resistor $R_d$ and the inductor $L_P$. FIG. 19 a schematic structural diagram of a transformer with resistors connected to inductors in parallel. As shown in FIG. 19, the damping resistors $R_d$ are connected in parallel with the inductors $L_P$, respectively. Each pair of the parallel-connected resistor $R_d$ and inductor $L_P$ is connected in series to the capacitors $C_P$, and then is connected in parallel with the corresponding secondary winding.

In an embodiment, the impedance element includes an inductor. Using the inductor instead of a resistor as the impedance element can avoid the problem that the voltage imbalance of the secondary winding deteriorates due to the inappropriate capacitance value of the capacitor. Meanwhile, the problem of increased loss caused by the resistor can be avoided.

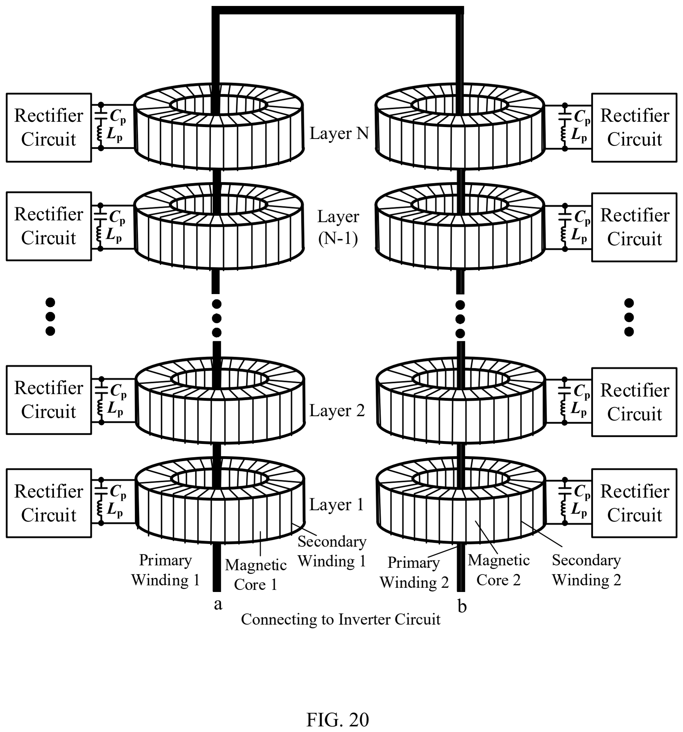

In an embodiment, the transformer includes a plurality of the rectifier circuits. The plurality of rectifier circuits are connected to the plurality of secondary windings, respectively. FIG. 20 is a schematic structural diagram of a transformer with resistors connected in series to inductors respectively, as shown in FIG. 20, the impedance element is configured as the inductors $L_P$. The capacitors $C_P$ are connected in series with the inductors $L_P$, respectively. Each pair of the series-connected capacitor $C_P$ and inductor $L_P$ serves as a whole. The two ends of the whole are connected to the two ends of the corresponding secondary winding. That is, the two ends of the whole are also connected to the two ends of the rectifier circuit, respectively.

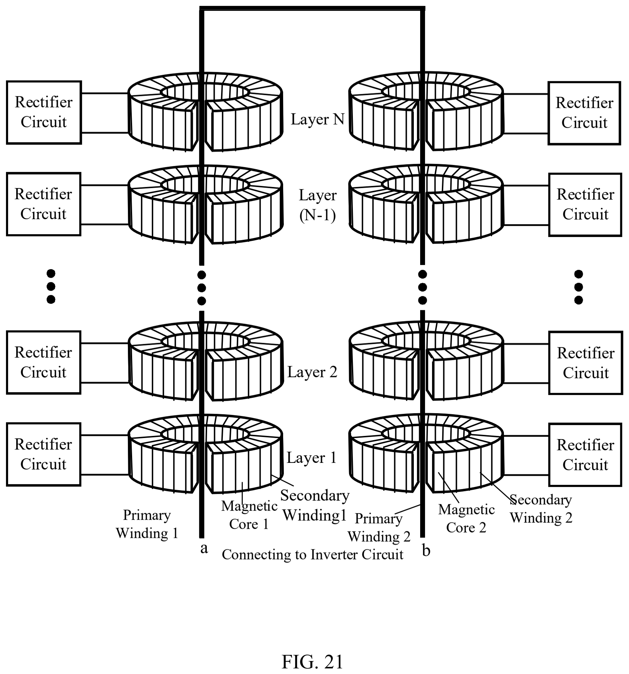

In an embodiment, FIG. 21 is a schematic diagram of a transformer having a magnetic core assembly with an air gap. As shown in FIG. 21, each of the magnetic core assemblies has an air gap of the same size. In addition, the transformer includes a plurality of the rectifier circuits. The plurality of rectifier circuits are connected to the plurality of secondary windings, respectively.

In an embodiment, each of the magnetic core assemblies has an air gap of the same size. The transformer includes a capacitor. The two ends of the capacitor are correspondingly connected to two ends of the secondary winding.

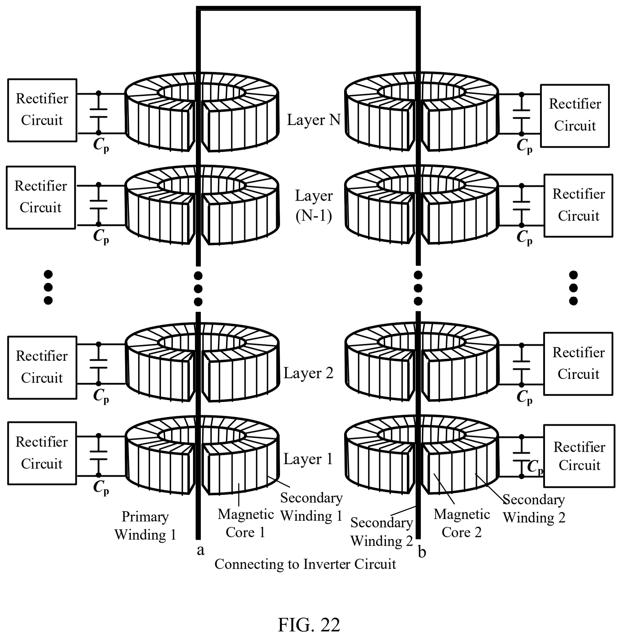

In an embodiment, the transformer includes a plurality of the rectifier circuits. As shown in FIG. 22, a transformer with magnetic core assemblies each having an air gap is provided, and each of the secondary windings is connected in parallel with a capacitor. By forming the air gap of the same size in each of the magnetic core assemblies, the equivalent permeabilities of the magnetic cores can be the same, and then the excitation inductances can also be the same, thereby ensuring the voltage balance of the secondary windings. Meanwhile, the capacitor can further compensate for the differences in the characteristics of the respective magnetic cores.

In an embodiment, each of the magnetic core assemblies has an air gap of the same size. The transformer includes a capacitor and an impedance element, and the impedance element is a resistor.

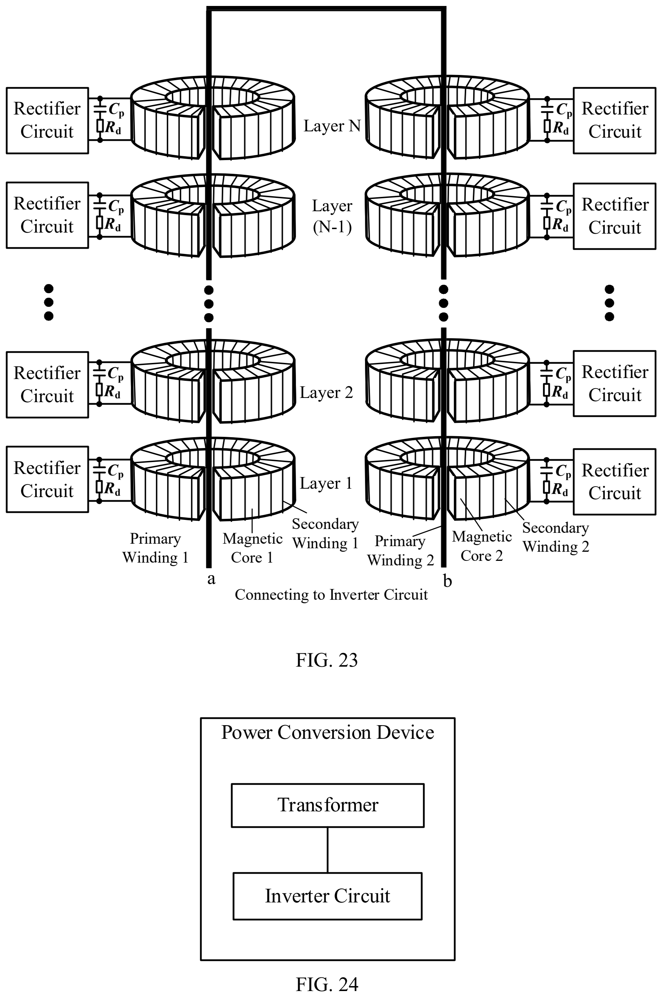

In an embodiment, the transformer further includes a plurality of rectifier circuits connected to the plurality of secondary windings respectively. FIG. 23 is a schematic diagram of a transformer having a magnetic core assembly with an air gap and a secondary winding connected to a capacitor and a resistor in parallel. A capacitor is connected in series with a resistor. The series-connected capacitor and resistor serve as a whole. The two ends of the whole are connected to the two ends of the corresponding secondary windings.

In an embodiment, each of the magnetic core assemblies has an air gap of the same size. The transformer includes a capacitor and an impedance element, the impedance element can also be a series-connected resistor and inductor, or the impedance element can also be an inductor.

Based on the same inventive concept, a power conversion device for implementing the above-mentioned transformer is provided in an embodiment of the present disclosure. The technical solutions for solving the problem of the power conversion device is similar to the technical solutions of the above power conversion method, so for the specific definitions of one or more power conversion device provided in the embodiments below, reference can be made to the definitions of the power conversion method above, which are not repeatedly described herein.

In an embodiment, as shown in FIG. 24, a power conversion device is provided. The power conversion device includes an inverter circuit and a transformer. The inverter circuit is connected to the transformer. Specifically, the inverter circuit can be connected to a primary winding of the transformer.

The transformer includes a primary winding, a plurality of magnetic core assemblies and a plurality of secondary windings. The primary winding passes through the plurality of magnetic core assemblies. The plurality of secondary windings are wound on the plurality of magnetic core assemblies, respectively. Each of the magnetic core assemblies is provided with an air gap of the same size. Alternatively, the transformer includes a capacitor and an impedance element. The capacitor is connected in series with the impedance element. The series-connected capacitor and impedance element serve as a whole. The two ends of the whole are connected to the two ends of the corresponding secondary winding, respectively.

In an embodiment, the transformer further includes a plurality of rectifier circuits. The plurality of rectifier circuits are connected to a plurality of secondary windings, respectively.

In an embodiment, the inverter circuit includes a switching element. The switching element adjusts the direction

**EP 4 765 174 A1**

of a current in the inverter circuit by changing an on state or an off state of the switching element. When each of the magnetic core assemblies has an air gap of the same size, although the air gap may lead to a decrease in the excitation inductance value and an increase in an excitation current, further increasing the conduction loss of a switching tube in the inverter circuit, the excitation current can assist the switching tube in turning on the Zero Voltage Switching (ZVS), thereby reducing the turn-on loss of the switching tube. Therefore, the total loss will not increase significantly.

In an embodiment, the impedance element includes a resistor. Alternatively, the impedance element includes a resistor and an inductor, and the resistor is connected in parallel with the inductor. Alternatively, the impedance element includes an inductor. The parameters of the impedance element are determined based on at least one of the following parameters: the frequency of the AC voltage input to the primary winding, the load range of the transformer, the characteristics of the magnetic core assemblies, or the preset loss of the transformer.

In an embodiment, each of the magnetic core assemblies has an air gap of the same size. The transformer includes a capacitor. The two ends of the capacitor are connected to the two ends of the corresponding secondary winding, respectively.

Based on the same inventive concept, a medical device for implementing the above-mentioned transformer is provided in an embodiment of the present disclosure. In an embodiment, a medical device is provided, and the medical device includes the transformer of the above embodiments.

In an embodiment, by providing the transformer, an electrical signal input to the medical device is subjected to voltage transformation and voltage stabilization. The medical device can be a Computed Tomography (CT) scanner, an X-ray machine, a radiotherapy machine, or any other device that needs to operate in a stable and safe power supply environment.

The transformer includes a primary winding, a plurality of magnetic core assemblies, and a plurality of secondary windings. The primary winding passes through the plurality of magnetic core assemblies. The plurality of secondary windings are wound on the plurality of magnetic core assemblies, respectively. Each of the magnetic core assemblies has an air gap of the same size. Alternatively, the transformer includes a capacitor and an impedance element. The capacitor is connected in series with the impedance element. The series-connected capacitor and impedance element serve as a whole. The two ends of the whole are connected to the two ends of the corresponding secondary winding, respectively. In an embodiment, the transformer further includes a plurality of rectifier circuits. The plurality of rectifier circuits are connected to the plurality of secondary windings, respectively.

In an embodiment, the impedance element includes a resistor. Alternatively, the impedance element includes a resistor and an inductor. The resistor is connected in parallel with the inductor. Alternatively, the impedance element includes an inductor. The parameters of the impedance element are determined based on at least one of the following parameters: the frequency of the AC voltage input to the primary winding, the load range of the transformer, the characteristics of the magnetic core assemblies, or the preset loss of the transformer.

In an embodiment, each of the magnetic core assemblies has an air gap of the same size. The transformer includes a capacitor. The two ends of the capacitor are connected to the two ends of the secondary winding, respectively.

In an embodiment, the inverter circuit includes a switching element. The switching element adjusts the direction of the current in the inverter circuit by changing the on state or the off state of the switching element.

The technical solutions for solving the problem of the medical device is similar to the technical solutions of the above power conversion method, so for the specific definitions of the medical device provided in one or more embodiments below, reference may be made to the definitions of the power conversion method above, which are not repeatedly described herein.

The transformer, the power conversion device, and the medical device can improve the differences in characteristics of the magnetic cores by forming the air gaps of the same size in the magnetic core assemblies in the transformer with a distributed structure, so as to achieve the same output voltages of the secondary windings of the transformer. Alternatively, by connecting the capacitor and the impedance element in parallel with the secondary winding, the capacitors can compensate for the differences in the characteristics of the magnetic cores, the impedance element can avoid the problem that the capacitors increase the degree of the voltage imbalance of the secondary windings at a specific frequency, thereby addressing the problem of the imbalance of the secondary voltages output by the plurality of secondary windings in the transformer.

**Claims**

1. A transformer, **characterized by** comprising:

    a primary winding;
    a plurality of magnetic core assemblies; and
    a plurality of secondary windings;

wherein the primary winding is configured to pass through the plurality of magnetic core assemblies;
the plurality of secondary windings are wound on the plurality of magnetic core assemblies, respectively; and
at least one of limitations is satisfied:

(a) each of the plurality of magnetic core assemblies having an air gap of the same size; or
(b) the transformer comprising a capacitor and an impedance element, wherein the capacitor is connected in series with the impedance element, the series-connected capacitor and impedance element are connected to two ends of a corresponding secondary winding.

2. The transformer according to claim 1, wherein the impedance element comprises a resistor.

3. The transformer according to claim 1, wherein the impedance element comprises a resistor and an inductor, and the resistor is connected in parallel with the inductor.

4. The transformer according to claim 1, wherein the impedance element comprises an inductor.

5. The transformer according to claim 1, wherein corresponding to the limitation of each of the plurality of magnetic core assemblies having the air gap of the same size, the transformer comprises a capacitor, and two ends of the capacitor are connected to two ends of a corresponding secondary winding.

6. The transformer according to any one of claims 1 to 5, wherein the transformer comprises a plurality of rectifier circuits, and the plurality of rectifier circuits are connected to the plurality of secondary windings, respectively.

7. The transformer according to any one of claims 1 to 4, wherein parameters of the impedance element are determined based on at least one of parameters of: a frequency of an alternating current (AC) voltage inputted to the primary winding, a load range of the transformer, differences in characteristics of the magnetic core assemblies, or a preset loss of the transformer.

8. The transformer according to any one of claims 1 to 7, wherein the air gap has the same length.

9. The transformer according to any one of claims 1 to 8, wherein winding directions of the secondary windings are the same, and numbers of turns of the secondary windings are the same.

10. The transformer according to claim 7, wherein the differences in the characteristics of the magnetic core assemblies comprise the differences in permeabilities and cross-sectional areas of magnetic cores.

11. The transformer according to claim 2, wherein a capacitance value of the capacitor is determined based on at least one of parameters of: a frequency of an AC voltage inputted to the primary winding, a load range of the transformer, differences in characteristics of the magnetic core assemblies, or a preset loss of the transformer.

12. A power conversion device, comprising an inverter circuit and the transformer according to any one of claims 1 to 11, wherein the inverter circuit is connected to the transformer.

13. The power conversion device according to claim 12, wherein the inverter circuit is connected to the primary winding of the transformer.

14. The power conversion device according to claim 12, wherein the inverter circuit comprises a switching element, and the switching element is configured to adjust a direction of a current in the inverter circuit by changing an on state or an off state of the switching element.

15. A medical device, comprising the transformer according to any one of claims 1 to 11.

FIG. 1

FIG. 2

FIG. 3

FIG. 4

FIG. 5

Rectifier Circuit

$C_p$

Rectifier Circuit

$C_p$

Rectifier Circuit

$C_p$

Rectifier Circuit

$C_p$

Layer N

Layer (N-1)

Layer 2

Layer 1

Rectifier Circuit

$C_p$

Rectifier Circuit

$C_p$

Rectifier Circuit

$C_p$

Rectifier Circuit

$C_p$

Primary Winding 1    Magnetic Core 1    Secondary Winding 1

Primary Winding 2    Magnetic Core 2    Secondary Winding 2

a    b

Connecting to Inverter Circuit

FIG. 6

FIG. 7

FIG. 8

FIG. 9

FIG. 10

18

FIG. 11

FIG. 12

FIG. 13

FIG. 14

FIG. 15

FIG. 16

FIG. 17

FIG. 18

Rectifier Circuit $C_p$ $L_p$ $R_d$ Layer N $C_p$ $L_p$ $R_d$ Rectifier Circuit

Rectifier Circuit $C_p$ $L_p$ $R_d$ Layer (N-1) $C_p$ $L_p$ $R_d$ Rectifier Circuit

Rectifier Circuit $C_p$ $L_p$ $R_d$ Layer 2 $C_p$ $L_p$ $R_d$ Rectifier Circuit

Rectifier Circuit $C_p$ $L_p$ $R_d$ Layer 1 $C_p$ $L_p$ $R_d$ Rectifier Circuit

Primary Winding 1  Magnetic Core 1  Secondary Winding1  Primary Winding 2  Magnetic Core 2  Secondary Winding 2

a  b

Connecting to Inverter Circuit

FIG. 19

Rectifier Circuit $C_p$ $L_p$ Layer N $C_p$ $L_p$ Rectifier Circuit

Rectifier Circuit $C_p$ $L_p$ Layer (N-1) $C_p$ $L_p$ Rectifier Circuit

Rectifier Circuit $C_p$ $L_p$ Layer 2 $C_p$ $L_p$ Rectifier Circuit

Rectifier Circuit $C_p$ $L_p$ Layer 1 $C_p$ $L_p$ Rectifier Circuit

Primary Winding 1    Magnetic Core 1    Secondary Winding 1    Primary Winding 2    Magnetic Core 2    Secondary Winding 2

a    Connecting to Inverter Circuit    b

FIG. 20

FIG. 21

FIG. 22

Rectifier Circuit

$C_p$
$R_d$

Layer N

$C_p$
$R_d$

Rectifier Circuit

Rectifier Circuit

$C_p$
$R_d$

Layer (N-1)

$C_p$
$R_d$

Rectifier Circuit

Rectifier Circuit

$C_p$
$R_d$

Layer 2

$C_p$
$R_d$

Rectifier Circuit

Rectifier Circuit

$C_p$
$R_d$

Layer 1

$C_p$
$R_d$

Rectifier Circuit

Primary Winding 1

Magnetic Core 1

Secondary Winding 1

Primary Winding 2

Magnetic Core 2

Secondary Winding 2

a

b

Connecting to Inverter Circuit

FIG. 23

Power Conversion Device

Transformer

Inverter Circuit

FIG. 24

**Europäisches Patentamt**
**European Patent Office**
**Office européen des brevets**

# EUROPEAN SEARCH REPORT

**Application Number**

EP 25 22 0828

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/142513 A1 (VINCIARELLI PATRIZIO [US]) 31 July 2003 (2003-07-31) * figures 9,15c,28,32,33 * * paragraph [0161] * * paragraph [0188] - paragraph [0189] * * paragraph [0211] - paragraph [0225] * | 1-15 | INV. H01F27/40 H01F3/14 H02M3/28 |
| X | US 2018/138801 A1 (CHEN RUNRUO [US]) 17 May 2018 (2018-05-17) * figures 1,2,6 * * paragraphs [0026], [0027], [0031], [0032], [0042], [0047] * | 1-14 | |
| X | DE 102 18 455 A1 (ABB PATENT GMBH [DE]) 6 November 2003 (2003-11-06) * figures 1,2,4 * * paragraph [0001] - paragraph [0008] * * paragraph [0022] - paragraph [0027] * | 1-8, 10-15 | |
| X | SU 266 037 A1 (INST POSTOYANNOGO TOKA [SU]) 15 July 1985 (1985-07-15) * abstract * * figure * | 1,2,5-7, 9-11 | **TECHNICAL FIELDS SEARCHED (IPC)** H01F H02M |

The present search report has been drawn up for all claims

| Place of search | Date of completion of the search | Examiner |
|---|---|---|
| Munich | 6 May 2026 | Tano, Valeria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 22 0828

06-05-2026

| Patent document cited in search report | | | Publication date | Patent family member(s) | | Publication date |
|---|---|---|---|---|---|---|
| US 2003142513 | A1 | | 31-07-2003 | DE | 03250638 T1 | 11-11-2004 |
| | | | | DE | 03250648 T1 | 11-11-2004 |
| | | | | EP | 1333553 A2 | 06-08-2003 |
| | | | | EP | 1333564 A2 | 06-08-2003 |
| | | | | EP | 1333565 A2 | 06-08-2003 |
| | | | | EP | 1333566 A2 | 06-08-2003 |
| | | | | EP | 1333567 A2 | 06-08-2003 |
| | | | | JP | 2004159485 A | 03-06-2004 |
| | | | | US | 2003142513 A1 | 31-07-2003 |
| US 2018138801 | A1 | | 17-05-2018 | CN | 110326207 A | 11-10-2019 |
| | | | | CN | 113258788 A | 13-08-2021 |
| | | | | EP | 3539206 A1 | 18-09-2019 |
| | | | | JP | 6985578 B2 | 22-12-2021 |
| | | | | JP | 2019534674 A | 28-11-2019 |
| | | | | US | 2018138801 A1 | 17-05-2018 |
| | | | | US | 2018278174 A1 | 27-09-2018 |
| | | | | WO | 2018089771 A1 | 17-05-2018 |
| DE 10218455 | A1 | | 06-11-2003 | DE | 10218455 A1 | 06-11-2003 |
| | | | | WO | 03092147 A1 | 06-11-2003 |
| SU 266037 | A1 | | 15-07-1985 | NONE | | |

EPO FORM P0459